# EUROPEAN PATENT APPLICATION

(11) **EP 1 582 528 A1**
(43) Date of publication of application: **05.10.2005**
(21) Application number: 03777243.1
(22) Date of filing: 04.12.2003
(51) Int. Cl.: C07H 19/073, C07H 19/173

(54) **PROCESS FOR PRODUCING PHOSPHOROAMIDITE**

(30) Priority: 09.12.2002 JP 2002357209
(71) Applicant: Mitsui Chemicals, Inc., Tokyo 105-7117 (JP)
(72) Inventor: KOMATSU, H., c/o Mitsui Chemicals, Inc., Sodegaura-shi, Chiba 299-0265 (JP); HIWARA, Mayumi, c/o Mitsui Chemicals, Inc., Sodegaura-shi, Chiba 299-0265 (JP)
(74) Representative: Paget, Hugh Charles Edward
(86) International application number: PCT/JP2003/015529
(87) International publication number: WO 2004/052908

(57) **Abstract**

The present invention provides high purity phosphoroamidite products by reducing the amount of impurities of triphosphite which has been additionally produced according to the conventional technique.

Phosphoroamidites can be obtained with high purity such that the excess reaction is suppressed by using a reaction activator hardly generating triphosphite.

According to the present invention, phosphoroamides can be obtained with high purity, which has heretofore been difficult to suppress the generation of impurities of triphosphite.

## Description

### TECHNICAL FIELD

The present invention relates to a method for preparing phosphoroamidite. More specifically, the invention relates to a reaction activator that is developed for enhancing the selectivity for generation of phosphoroamidite by reducing the amount of the generated by-product comprising triphosphite represented by the general formula [5], in preparation of phosphoroamidite represented by the general formula [4], 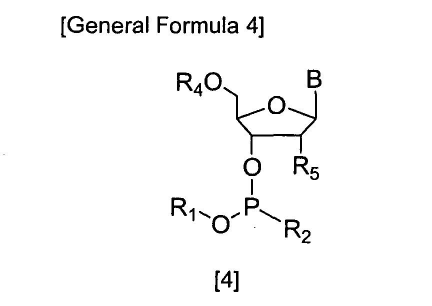 wherein R₁ represents an alkyl group having 1 to 4 carbon atoms, an alkyl group having 1 to 4 carbon atoms substituted by a cyano group or an alkyl group having 1 to 4 carbon atoms substituted by a silyl group; R₂ represents an amino group substituted by an alkyl group having 2 to 5 carbon atoms or an alicyclic amino group having 4 to 7 carbon atoms; R₄ represents a protecting group of a hydroxyl group; R₅ represents a hydrogen atom, a halogen atom, an alkyl group having 1 to 4 carbon atoms or a substituted hydroxyl group; and B represents a nucleic acid base or a protected nucleic acid base, 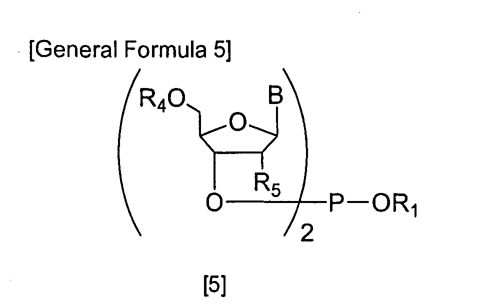 wherein R₁, R₄ and R₅ represent the same as those described above.

### BACKGROUND ART

In recent years, with the process of genome-based drug discovery, antisense DNA drugs and the like have been rapidly developed. The demand for DNA oligomer as a raw material and phosphoroamidites as a raw material of oligomer has become higher. In order to use phosphoroamidites for the purpose of medical supplies, it needs to manufacture products with very high purity.

Phosphoroamidites can heretofore be synthesized by a method as described in, for example, Nucleic Acids Res, 12, p. 4051 (1984) [or Nucleic Acids Res, 18, p. 177 (1987)] comprising reacting phosphorodiamidite with a hydroxyl group of nucleoside using a tetrazole reagent as a reaction activator. In this report, impurities generated by the reaction are analyzed in detail. Of major impurities, a decomposed product caused by water can be decreased by reducing the amount of water in the reaction system. However, it is difficult to decrease the by-product generated by an action of an activator used for the reaction. As shown in the formula 1, 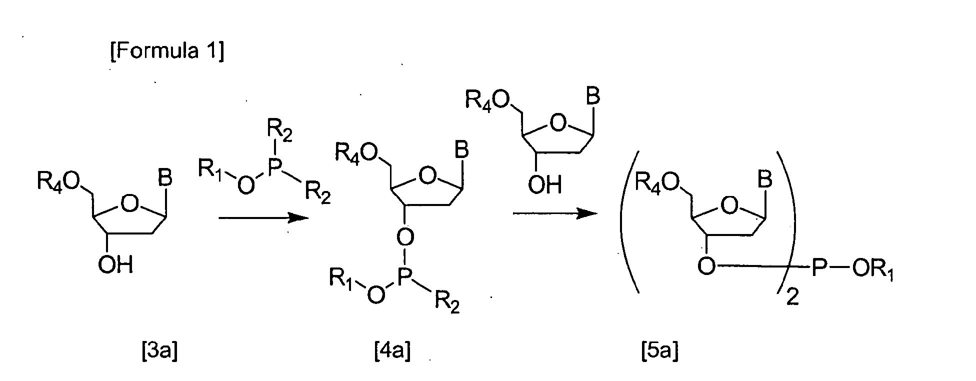 wherein R₁ represents a methyl group or a cyanoethyl group; R₂ represents a diisopropylamino group; R₄ represents a 4,4'-dimethoxytrityl group; and B represents a 1-thymine group,
an over-reaction product (triphosphite represented by the general formula [5a]) is generated by further reacting phosphoroamidite represented by the general formula [4a] to be targeted with a raw material nucleoside represented by the general formula [3a]. A mixture ratio is described as low, i.e., 1% (NMR analysis result), whereas it is required to be further decreased for the purpose of medical supplies such as antisense drugs or the like. Phosphoroamidites are unstable so that it is difficult to do purification. Accordingly, it is necessary to reduce the by-product in a reaction step. As described in US Patent No. 4,415,732, tetrazole is originally developed for the purpose of synthesis of triphosphite such as oligomer so that the reaction reaching triphosphite represented by the general formula [5a] from phosphoroamidite represented by the general formula [4a] is very fast. At present, tetrazole is the mostly used activator for the synthesis of oligomer.

In a method described in Org. Process Res. Dev. 4, p. 175 (2000) or WO99/62922, in order to overcome such a defect of high cost of tetrazole, pyridine-trifluoro acetate is used. However, as the usefulness of oligomer synthesis like tetrazole is described, the formation potential of triphosphite is high.

For the purpose of use in oligomer synthesis, many activators for replacing tetrazole have been reported. However, in order to conduct the oligomer synthesis in a solid phase synthesis, it was required to form triphosphite in a short time if at all possible. From such an aspect, its purpose was different from a selective synthesis of phosphoroamidite. Namely, in order to prepare phosphoroamidite represented by the general formula [4a], it was required to decrease the amount of by-product of triphosphite represented by the general formula [5a] so that these methods are not appropriate. A typical example has been described in Tetrahedron Lett. (24), p. 3171 (1983). 5-(4-nitrophenyl)tetrazole reported in this report is designed to greatly exceed the reaction rate of tetrazole. Thus, oligomer, i.e., triphosphite, is quite efficiently formed. Furthermore, phenyltetrazole was also exemplified as an activator for the triphosphite synthesis in the drawing. However, there was a determination describing that an evaluation of its activity was of no value as it had a bad solubility. It failed to find its usefulness as an activator capable of selectively synthesizing phosphoroamidite.

In this manner, an activator capable of selectively synthesizing phosphoroamidite represented by the general formula [4a] while hardly capable of proceeding the reaction to triphosphite represented by the general formula [5a] has not been developed.

### DISCLOSURE OF THE INVENTION

In consideration of the conventional problems, an object of the present invention is to provide a method for preparing phosphoroamidite in which the side reaction is effectively small.

In order to achieve the above object, as a result of an extensive study, the present inventors have found that 5-phenyltetrazole has generated phosphoroamidite with a good selectivity. Thus, the present invention has been completed.

That is, the present invention is specified by the matters described in the following (1) to (5).
(1) A method for preparing phosphoroamidite with a reagent of a compound represented by the general formula [1], 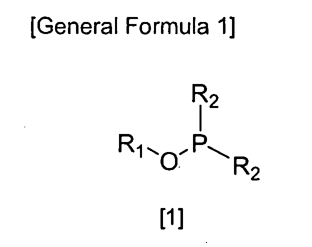 wherein R₁ represents an alkyl group having 1 to 4 carbon atoms, an alkyl group having 1 to 4 carbon atoms substituted by a cyano group or an alkyl group having 1 to 4 carbon atoms substituted by a silyl group; and R₂ represents an amino group substituted by an alkyl group having 2 to 5 carbon atoms or an alicyclic amino group having 4 to 7 carbon atoms,
   wherein a substituted tetrazole represented by the general formula [2] is used as a reaction activator, 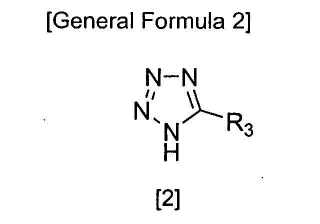 wherein R₃ represents an alicyclic alkyl group having 1 to 6 carbon atoms, an aryl group substituted by an alkyl group having 1 to 4 carbon atoms or an unsubstituted aryl group.
(2) The preparation method as described in (1), wherein generation of triphosphite represented by the general formula [5] is suppressed,
   when phosphoroamidite represented by the general formula [4] is synthesized by using a nucleoside derivative represented by the general formula [3] as a raw material, 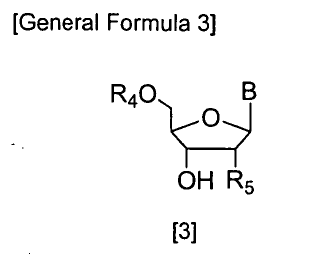 wherein R₄ represents a protecting group of a hydroxyl group; R₅ represents a hydrogen atom, a halogen atom, an alkyl group having 1 to 4 carbon atoms or a substituted hydroxyl group; and B represents a nucleic acid base or a protected nucleic acid base, 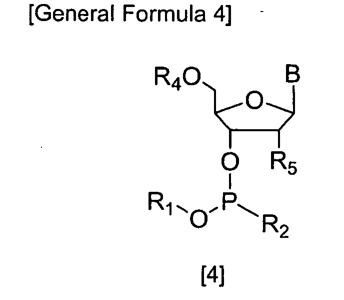 wherein R₁, R₂, R₄, R₅ and B represent the same as those described above, 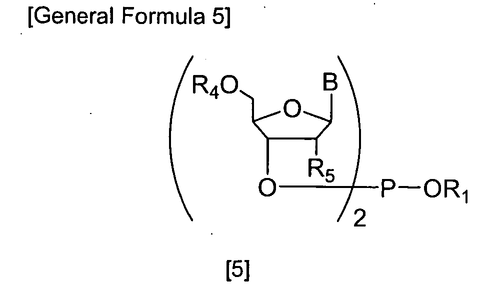 wherein R₁, R₂, R₄ and R₅ represent the same as those described above.
(3) The preparation method as described in (1) or (2), wherein R₃ in the general formula [2] is a phenyl group.
(4) The preparation method as described in any one of (1) to (3), wherein, in the general formula [1], R₁ is a cyanoethyl group and R₂ is a diisopropylamino group.
(5) The preparation method as described in any one of (2) to (4), wherein, in the general formulae [3] and [4], R₄ is a 4,4'-dimethoxytrityl group, R₅ is a hydrogen atom and B is a 1-thymine group, an N4-benzoyl-1-cytosine group, an N6-benzoyl-9-adenine group or an N2-isobutyryl-9-guanine group.

According to the present invention, it has become possible to prepare high purity phosphoroamidites effectively, as compared to the conventional process, by using a method capable of mass production.

### BEST MODE FOR CARRYING OUT THE INVENTION

The present invention will be described in more detail below.

In the phosphoroamidite represented by the general formula [1] or the like, as an alkyl group having 1 to 4 carbon atoms in R₁, there can be exemplified, for example, a methyl group, an ethyl group, an n-propyl group, an isopropyl group, a cyclopropyl group, an n-butyl group, a sec-butyl group, an isobutyl group, a tert-butyl group and the like. Examples of an alkyl group having 1 to 4 carbon atoms substituted by a cyano group include, though not restricted to, a 2-cyanoethyl group, a 2-cyanopropyl group, a 2-cyanocyclopropyl group, a 2-cyanobutyl group, a 2-cyano-2-methylpropyl group and the like. Examples of an alkyl group having 1 to 4 carbon atoms substituted by a silyl group in R₁ include, though not restricted to, a 2-trimethylsilylethyl group, a 2-trimethylsilylpropyl group, a 2-trimethylsilylbutyl group, a 2-trimethylsilyl-2-methylpropyl group, a 2-triethylsilylethyl group, a 2-triethylsilylpropyl group, a 2-triethylsilylbutyl group, a 2-triethylsilyl-2-methylpropyl group, a 2-triisopropylbutyl group, a 2-triisopropylsilyl-2-methylpropyl group, a 2-triisopropylsilylethyl group, a 2-triisopropylsilylpropyl group, a 2-triisopropylsilylbutyl group, a 2-triisopropylsilyl-2-methylpropyl group, a 2-isopropylbutyl group, a 2-triisopropylsilyl-2-methylpropyl group, a 2-(tert-butylmethylsilyl)ethyl group, a 2-(tert-butyldimethylsilyl)propyl group, a 2-(tert-butyldimethylsilyl)butyl group, a 2-(tert-butyldimethylsilyl)-2-methylpropyl group and the like. Examples of an amino group substituted by an alkyl group having 2 to 5 carbon atoms in R₂ include, though not restricted to, a diethylamino group, an ethyl(isopropyl)amino group, a di(n-propyl)amino group, a diisopropylamino group, a dicyclopropylamino group, a di(n-butyl)amino group, a di(sec-butyl)amino group, a di(isobutyl)amino group, a di(tert-butyl)amino group, a di(n-pentyl)amino group, a di(isopentyl)amino group, a di(neopentyl)amino group, a dicyclopentyl amino group and the like. Examples of an alicyclic amino group having 4 to 7 carbon atoms in R₂ include, though not restricted to, a pyrrolidino group, a 2-methylpyrrolidino group, a 2,4-dimethylpyrrolidino group, a piperidino group, a heptamethyleneimino group and the like.

In a substitute tetrazole represented by the general formula [2] or the like, as an alicyclic alkyl group having 1 to 6 carbon atoms in R₃, there can be exemplified, for example, a cyclopropyl group, a cyclobutyl group, a cyclopentyl group, a cyclohexyl group and the like. An aryl group substituted by an alkyl group having 1 to 4 carbon atoms in R₃ represents an aryl group substituted by a methyl group, an ethyl group, an n-propyl group, an isopropyl group, an n-butyl group, a sec-butyl group, an isobutyl group or a tert-butyl group, and examples thereof include, though not restricted to, a 2-methylphenyl group, a 2-ethylphenyl group, a 2-isopropylphenyl group, a 2-(2-butyl)phenyl group, a 2-(tert-butyl)phenyl group, a 3-methylphenyl group, a 3-ethylphenyl group, a 3-isopropylphenyl group, a 3-(2-butyl)phenyl group, a 3-(tert-butyl)phenyl group, a 4-methyl phenyl group, a 4-ethylphenyl group, a 4-isopropylphenyl group, a 4-(2-butyl)phenyl group, a 4-(tert-butyl)phenyl group, a 2,6-dimethylphenyl group, a 2,6-diethylphenyl group, a 2,6-diisopropylphenyl group, a 2,6-bis(2-butyl)phenyl group, a 2,6-bis(tert-butyl)phenyl group, a 3-methyl-2-furfuryl group, a 3-ethyl-2-furfuryl group, a 3-isopropyl-2-furfuryl group, a 3-(2-butyl)-2-furfuryl group, a 3-(tert-butyl)-2-furfuryl group, a 4-methyl-2-furfuryl group, a 4-ethyl-2-furfuryl group, a 4-isopropyl-2-furfuryl group, a 4-(2-butyl)-2-furfuryl group, a 4-(tert-butyl)-2-furfuryl group, a 3,5-dimethyl-2-furfuryl group and the like. Examples of an unsubstituted aryl group in R₃ include, though not restricted to, a phenyl group, a 2-furfuryl group, a 3-furfuryl group, a 2-thiophenyl group, a 3-thiophenyl group and the like. 5-phenyl-1H-tetrazole is particularly preferable for a substituted tetrazole represented by the general formula [2]. This substituted tetrazole may be added to the reaction with a base as a salt or may be added as it is. A base forming a salt is preferably an organic base, and examples thereof includes, though not restricted to, tertiary amines such as triethylamine, ethyldiisopropylamine, tri(n-butyl)amine, 1-methylpiperidine, 1-methylpyrrolidine and the like or secondary amines such as diethylamine, diisopropylamine, di(n-butyl)amine, pyrrolidine, piperidine and the like.

In a nucleoside derivative represented by the general formula [3], examples of a protecting group of a hydroxyl group in R₄ include alkyl groups such as a methyl group, an ethyl group, an isopropyl group, an n-butyl group, an i-butyl group, a t-butyl group, a pentyl group, a benzyl group, a 2-methoxybenzyl group, a 3-methoxybenzyl group, a 4-methoxybenzyl group, a 2-methylbenzyl group, a 3-methylbenzyl group, a 4-methylbenzyl group, a methoxyethyl group, an ethoxyethyl group, a benzyloxymethyl group, a benzyloxyethyl group, an acetoxymethyl group, an acetoxyethyl group, a benzoyloxymethyl group, a benzoyloxyethyl group, a methoxyethoxyethyl group, a propargyl group, an allyl group and the like or an alkyl group further having a substitute at the alkyl group; aryl groups such as a phenyl group, a 2-methoxyphenyl group, a 3-methoxyphenyl group, a 4-methoxyphenyl group, a 4-phenylphenyl group, a 2-pyridinyl group, a 3-pyridinyl group, a 4-pyridinyl group and the like; acyl groups such as a formyl group, an acetyl group, a propionyl group, a benzoyl group, a 2-methoxybenzoyl group, a 3-methoxybenzoyl group, a 4-methoxybenzoyl group, a 2-methylbenzoyl group, a 3-methylbenzoyl group, a 4-methylbenzoyl group, a 2-nitrobenzoyl group, a 3-nitrobenzoyl group, a 4-nitrobenzoyl group, a 4-phenylbenzoyl group, a 2-chlorobenzoyl group, a 3-chlorobenzoyl group, a 4-chlorobenzoyl group and the like; urethane groups such as an aminocarbonyl group, a dimethylaminocarbonyl group, a methylaminocarbonyl group, an ethylaminocarbonyl group, a diethylaminocarbonyl group, a phenylaminocarbonyl group and the like; sulfonate groups such as a methanesulfonyl group, an ethanesulfonyl group, a benzenesulfonyl group, a 2-methylbenzenesulfonyl group, a 3-methylbenzenesulfonyl group, a 4-methylbenzenesulfonyl group, a trifluoromethanesulfonyl group, a trichloromethanesulfonyl group and the like; silyl groups such as a trimethylsilyl group, a triethylsilyl group, a t-butyldimethylsilyl group, a t-butyldiphenylsilyl group and the like; and trityl groups such as a trityl group, a 4-methoxytrityl group, a 4,4'-dimethoxytrityl group and the like.

A halogen atom in R₅ represents a fluorine atom, a chlorine atom, a bromine atom or an iodine atom. Examples of an alkyl group having 1 to 4 carbon atoms in R₅ include a methyl group, an ethyl group, an n-propyl group, an isopropyl group, a cylcopropyl group, an n-butyl group, a sec-butyl group, an isobutyl group, a tert-butyl group and the like.

A substituted hydroxyl group in R₅ represents a hydroxyl group substituted by a substituent capable of being a protecting group of a hydroxyl group in general such as carboxylic acid ester, sulfonate ester, ether, urethane, a silyl group and the like. Examples of a protecting group of a hydroxyl group include alkyl groups such as a methyl group, an ethyl group, an isopropyl group, an n-butyl group, an i-butyl group, a t-butyl group, a pentyl group, a benzyl group, a 2-methoxybenzyl group, a 3-methoxybenzyl group, a 4-methoxybenzyl group, a 2-methylbenzyl group, a 3-methylbenzyl group, a 4-methylbenzyl group, a methoxyethyl group, an ethoxyethyl group, a benzyloxymethyl group, a benzyloxyethyl group, an acetoxymethyl group, an acetoxyethyl group, a benzoyloxymethyl group, a benzoyloxyethyl group, a methoxyethoxyethyl group, a propargyl group, an allyl group and the like or an alkyl group further having a substitute at the alkyl group; aryl groups such as a phenyl group, a 2-methoxyphenyl group, a 3-methoxyphenyl group, a 4-methoxyphenyl group, a 4-phenylphenyl group, a 2-pyridinyl group, a 3-pyridinyl group, a 4-pyridinyl group and the like; acyl groups such as a formyl group, an acetyl group, a propionyl group, a benzoyl group, a 2-methoxybenzoyl group, a 3-methoxybenzoyl group, a 4-methoxybenzoyl group, a 2-methylbenzoyl group, a 3-methylbenzoyl group, a 4-methylbenzoyl group, a 2-nitrobenzoyl group, a 3-nitrobenzoyl group, a 4-nitrobenzoyl group, a 4-phenylbenzoyl group, a 2-chlorobenzoyl group, a 3-chlorobenzoyl group, a 4-chlorobenzoyl group and the like; urethane groups such as an aminocarbonyl group, a dimethylaminocarbonyl group, a methylaminocarbonyl group, an ethylaminocarbonyl group, a diethylaminocarbonyl group, a phenylaminocarbonyl group and the like; sulfonate groups such as a methanesulfonyl group, an ethanesulfonyl group, a benzenesulfonyl group, a 2-methylbenzenesulfonyl group, a 3-methylbenzenesulfonyl group, a 4-methylbenzenesulfonyl group, a trifluoromethanesulfonyl group, a trichloromethanesulfonyl group and the like; silyl groups such as a trimethylsilyl group, a triethylsilyl group, a t-butyldimethylsilyl group, a t-butyldiphenylsilyl group and the like.

Examples of a substituted hydroxyl group in R₅ include a methoxy group, an ethoxy group, an isopropyloxy group, an n-butyloxy group, an i-butyloxy group, a t-butyloxy group, a pentyloxy group, a benzyloxy group, a 2-methoxybenzyloxy group, a 3-methoxybenzyloxy group, a 4-methoxybenzyloxy group, a 2-methylbenzyloxy group, a 3-methylbenzyloxy group, a 4-methylbenzyloxy group, a methoxyethyloxy group, an ethoxyethyloxy group, a benzyloxymethoxy group, a benzyloxyethoxy group, an acetoxymethoxy group, an acetoxyethoxy group, a benzoyloxymethoxy group, a benzoyloxyethoxy group, a methoxyethoxyethoxy group, a propargyloxy group, an allyloxy group, a phenyloxy group, a 2-methoxyphenyloxy group, a 3-methoxyphenyloxy group, a 4-methoxyphenyloxy group, a 4-phenylphenyloxy group, a 2-pyridinyloxy group, a 3-pyridinyloxy group, a 4-pyridinyloxy group, a formyloxy group, an acetyloxy group, a propionyloxy group, a benzoyloxy group, a 2-methoxybenzoyloxy group, a 3-methoxybenzoyloxy group, a 4-methoxybenzoyloxy group, a 2-methylbenzoyloxy group, a 3-methylbenzoyloxy group, a 4-methylbenzoyloxy group, a 2-nitrobenzoyloxy group, a 3-nitrobenzoyloxy group, a 4-nitrobenzoyloxy group, a 4-phenylbenzoyloxy group, a 2-chlorobenzoyloxy group, a 3-chlorobenzoyloxy group, a 4-chlorobenzoyloxy group, an aminocarbonyloxy group, a dimethylaminocarbonyloxy group, a methylaminocarbonyloxy group, an ethylaminocarbonyloxy group, a diethylaminocarbonyloxy group, a phenylaminocarbonyloxy group, a methanesulfonyloxy group, an ethanesulfonyloxy group, a benzenesulfonyloxy group, a 2-methylbenzenesulfonyloxy group, a 3-methylbenzenesulfonyloxy group, a 4-methylbenzenesulfonyloxy group, a trifluoromethanesulfonyloxy group, a trichloromethanesulfonyloxy group, a trimethylsilyloxy group, a triethylsilyloxy group, a t-butyldimethylsilyloxy group, a t-butyldiphenylsilyloxy group and the like.

A nucleic acid base in B may be a natural or unnatural nucleic acid base and examples thereof include, though not restricted to, a 1-thymine group, a 1-cytosine group, a 9-adenine group, a 9-guanine group, a 1-uracil group, a 5-fluoro-1-uracil group, a 5-trifluoro-1-thymine group, a 5-methyl-1-cytosine group, a 6-chloro-9-purine group, a 2,6-diamino-9-purine group, a 7-adenine group, a 7-guanine group, an 8-bromo-9-adenine group, an 8-bromo-9-guanine group, a 2-amino-9-purine group, an 8-aza-9-adenine group, an 8-aza-9-guanine group, an 8-deaza-9-adenine group, an 8-deaza-9-guanine group and the like.

For a protected nucleic acid base in B, either of a natural or unnatural nucleic acid base may be protected. The protected nucleic acid base is not particularly restricted as far as it can be usually used as a protecting group. Examples of a protecting group include an acetyl group, an isobutyryl group, a benzoyl group, a dimethylamino methylidyne group, a dibutylamino methylidyne group, a palmitoyl group, a benzyloxyacetyl group and the like. Examples of a protected nucleic acid base include an N4-benzoyl-1-cytosine group, an N4-acetyl-1-cytosine group, an N6-benzoyl-9-adenine group, an N2-isobutyryl-9-guanine group and the like.

A reaction solvent used in the method of the present invention is not particularly restricted as far as it does not influence on the reaction. The reaction solvent is preferably an aprotic solvent other than alcohols. Examples thereof include acetonitrile, dichloromethane, chloroform, THF, dimethoxyethane, ethyl acetate, butyl acetate, hexane, cyclohexane, toluene and the like. The mixed solvents thereof can also be used.

In the method of the present invention, a reaction temperature can be from -30°C to the boiling point of a solvent in use. In particular, it is preferably from -10°C to 40°C.

The amount of a reagent used is not particularly restricted as far as it does not influence on the work-up procedure. Considering the economical efficiency, the amount is preferably from 0.8 to 1.5 equivalent based on the molar amount of a raw material.

The amount of a reaction activator used is preferably from 0.01 to 2 equivalents based on the molar amount of a raw material. The amount of from 0.1 to 1.5 equivalent is particularly preferable.

### EXAMPLES

The present invention is described specifically below by way of Examples. However, the present invention is not restricted to these Examples.

### Example 1

### Synthesis of 5'-O-(4,4'-dimethoxytrityl)thymidine 3'-O-(2-cyanoethyl N,N-diisopropylphosphoroamidite)

2.0 g of 5'-O-(4,4'-dimethoxytrityl)thymidine (containing 0.5 equivalent of 4-methyl-2-pentanone) was mixed with 10 mL of dehydrated acetonitrile and 1.22 g of 2-cyanoethyl N,N,N',N'-tetraisopropylphosphorodiamidite (1.2 equivalent to the molar number of a raw material) was dropped to a suspension stirred at a room temperature, followed by further stirring. Then, 0.05 g of 5-phenyl-1 H-tetrazole (0.1 equivalent to the molar number of a raw material) was added thereto and the resulting mixture was stirred at a room temperature for 8 hours.

The reaction solution was analyzed according to the high performance liquid chromatography (reverse phase column, eluent: water/acetonitrile 5/5 (TEAA 250 mM), detection wavelength: 254 nm). As a result, the yield was 97%. The reaction selectivity (HPLC area % of the entitled compound / HPLC area % of the by-product) represented by the ratio of the entitled compound to the by-product represented by the general formula [5b] was 451, 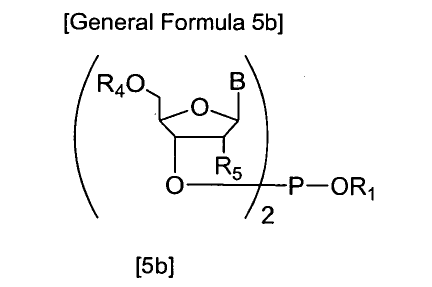 wherein R₁ represents a 2-cyanoethyl group; R₄ represents a 4,4'-dimethoxytrityl group; R₅ represents a hydrogen atom; and B represents 1-thimine.

### Comparative Examples 1 and 2

### Synthesis of 5'-O-(4,4'-dimethoxytrityl)-2'-deoxythymidine 3'-O-(2-cyanoethyl N,N-diisopropylphosphoroamidite)

The reaction was conducted in the same manner as in Example 1, except that 0.024 g of tetrazole (0.1 equivalent to the molar number of a raw material: Comparative Example 1) and 0.068 g of pyridinium trifluoroacetate (0.1 equivalent to the molar number of a raw material: Comparative Example 2) were respectively used, instead of 5-phenyl-1 H-tetrazole. The reaction solution after 8 or 24 hours was analyzed according to the high performance liquid chromatography (reverse phase column, eluent: water/acetonitrile 5/5 (TEAA 250 mM), detection wavelength: 254 nm). The results are shown in Table 1.

**[Table 1]**

| | Example 1 | Comparative Example 1 | Comparative Example 2 |
|---|---|---|---|
| Reaction Selectivity (after 8 hours) | 451 | 265 | 281 |
| Yield (%, after 8 hours) | 94 | 94 | 64 |
| Reaction Selectivity (after 24 hours) | - | - | 192 |
| Yield (%, after 24 hours) | - | - | 82 |

In the above Table 1, the reaction selectivity is the ratio of HPLC area % of the entitled compound to HPLC area % of the by-product represented by the general formula [5b] (HPLC area % of the entitled compound / HPLC area % of the by-product represented by the general formula [5b]). In the formula [5b] of the by-product, R₁ represents a 2-cyanoethyl group, R₄ represents a 4,4'-dimethoxytrityl group, R₅ represents a hydrogen atom and B represents 1-thymine.

### Example 2

### Synthesis of 5'-O-(4,4'-dimethoxytrityl)-N6-benzoyl-2'-deoxyadenosine 3'-O-(2-cyanoethyl N,N-diisopropylphosphoroamidite)

2.0 g of 5'-O-(4,4'-dimethoxytrityl)-N6-benzoyl-2'-deoxyadenosine was mixed with 10 mL of dehydrated acetonitrile and 1.10 g of 2-cyanoethyl N,N,N',N'-tetraisopropylphosphorodiamidite (1.2 equivalent to the molar number of a raw material) was dropped to a suspension stirred at a room temperature, followed by further stirring. Then, 0.53 g of 5-phenyl-1H-tetrazole (1.2 equivalent to the molar number of a raw material) was added thereto and the resulting mixture was stirred at a room temperature for 2 hours.

The reaction solution was analyzed according to the high performance liquid chromatography (reverse phase column, eluent: water/acetonitrile 5/5 (TEAA 250 mM), detection wavelength: 254 nm). As a result, the yield was 96%. The reaction selectivity (HPLC area % of the entitled compound / HPLC area % of the by-product) represented by the ratio of the entitled compound to the by-product represented by the general formula [5c] was 152, 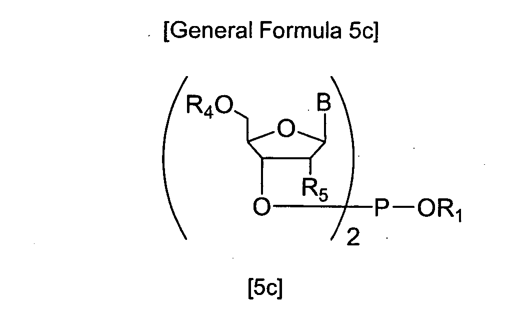 wherein R₁ represents a 2-cyanoethyl group; R₄ represents a 4,4'-dimethoxytrityl group; R₅ represents a hydrogen atom; and B represents N6-benzoyl-9-adenine.

Further, the reaction solution after 96 hours was analyzed. As a result, the yield was 99% and the reaction selectivity was 187.

### Example 3

### Synthesis of 5'-O-(4,4'-dimethoxytrityl)-N6-benzoyl-2'-deoxyadenosine 3'-O-(2-cyanoethyl N,N-diisopropylphosphoroamidite)

The reaction was conducted in the same manner as in Example 2, except that the equivalent of 5-phenyl-1H-tetrazole was changed. 0.04 g of 5-phenyl-1 H-tetrazole (0.1 equivalent to the molar number of a raw material) was used and the resulting mixture was stirred for 24 hours.

The reaction solution was analyzed according to the high performance liquid chromatography (reverse phase column, eluent: water/acetonitrile 5/5 (TEAA 250 mM), detection wavelength: 254 nm). As a result, the yield was 99%. The reaction selectivity (HPLC area % of the entitled compound / HPLC area % of the by-product) represented by the ratio of the entitled compound to the by-product represented by the general formula [5c] was 699.

### Example 4

### Synthesis of 5'-O-(4,4'-dimethoxytrityl)-N6-benzoyl-2'-deoxyadenosine 3'-O-(2-cyanoethyl N,N-diisopropylphosphoroamidite)

The reaction was conducted in the same manner as in Example 2, except that the equivalent of 5-phenyl-1 H-tetrazole was changed. 0.22 g of 5-phenyl-1 H-tetrazole (0.5 equivalent to the molar number of a raw material) was used and the resulting mixture was stirred for 8 hours.

The reaction solution was analyzed according to the high performance liquid chromatography (reverse phase column, eluent: water/acetonitrile 5/5 (TEAA 250 mM), detection wavelength: 254 nm). As a result, the yield was 99%. The reaction selectivity (HPLC area % of the entitled compound / HPLC area % of the by-product) represented by the ratio of the entitled compound to the by-product represented by the general formula [5c] was 785.

### Comparative Examples 3 to 5

The reaction was conducted in the same manner as in Example 2, except that 0.256 g of tetrazole (1.2 equivalent to the molar number of a raw material: Comparative Example 3), 0.707 g of pyridinium trifluoroacetate (1.2 equivalent to the molar number of a raw material. Comparative Example 4) and 0.689 g of 5-(4-nitrophenyl)-1H-tetrazole (1.2 equivalent to the molar number of a raw material: Comparative Example 5) were respectively used, instead of 5-phenyl-1 H-tetrazole. The reaction solution after 96 hours was analyzed according to the high performance liquid chromatography (reverse phase column, eluent: water/acetonitrile 5/5 (TEAA 250 mM), detection wavelength: 254 nm). The results are shown in Table 2.

**[Table 2]**

| | Example 2 | Comparative Example 3 | Comparative Example 4 | Comparative Example 5 |
|---|---|---|---|---|
| Reaction Selectivity | 187 | 49 | 91 | 9 |
| Yield (%) | 99 | 99 | 92 | 79 |

### Comparative Examples 6 to 8

The reaction was conducted in the same manner as in Example 4, except that 0.107 g of tetrazole (0.5 equivalent to the molar number of a raw material: Comparative Example 6), 0.295 g of pyridinium trifluoroacetate (0.5 equivalent to the molar number of a raw material: Comparative Example 7) and 0.291 g of 5-(4-nitrophenyl)-1H-tetrazole (0.5 equivalent to the molar number of a raw material: Comparative Example 8) were respectively used, instead of 5-phenyl-1 H-tetrazole. The reaction solution after 96 hours was analyzed according to the high performance liquid chromatography (reverse phase column, eluent: water/acetonitrile 5/5 (TEAA 250 mM), detection wavelength: 254 nm). The results are shown in Table 3.

**[Table 3]**

| | Example 4 | Comparative Example 6 | Comparative Example 7 | Comparative Example 8 |
|---|---|---|---|---|
| Reaction Selectivity | 785 | 101 | 39 | 153 |
| Yield (%) | 99 | 99 | 92 | 98 |

In the above Table 3, the reaction selectivity is the ratio of HPLC area % of the entitled compound to HPLC area % of the by-product represented by the general formula [5c] (HPLC area % of the entitled compound / HPLC area % of the by-product represented by the general formula [5c]).

### Example 5

### Synthesis of 5'-O-(4,4'-dimethoxytrityl)-N4-benzoyl-2'-deoxycytidine 3'-O-(2-cyanoethyl N,N-diisopropylphosphoroamidite)

2.0 g of 5'-O-(4,4'-dimethoxytrityl)-N4-benzoyl-2'-deoxycytidine was mixed with 10 mL of dehydrated acetonitrile and 1.14 g of 2-cyanoethyl N,N,N',N'-tetraisopropylphosphorodiamidite (1.2 equivalent to the molar number of a raw material) was dropped to a suspension stirred at a room temperature, followed by further stirring. Then, 0.185 g of 5-phenyl-1 H-tetrazole (0.4 equivalent to the molar number of a raw material) was added thereto and the resulting mixture was stirred at a room temperature for 5 hours.

The reaction solution was analyzed according to the high performance liquid chromatography (reverse phase column, eluent: water/acetonitrile 4/6 (TEAA 62.5 mM), detection wavelength: 254 nm). As a result, the yield was 99%. The reaction selectivity (HPLC area % of the entitled compound / HPLC area % of the by-product) represented by the ratio of the entitled compound to the by-product represented by the general formula [5d] was 284, 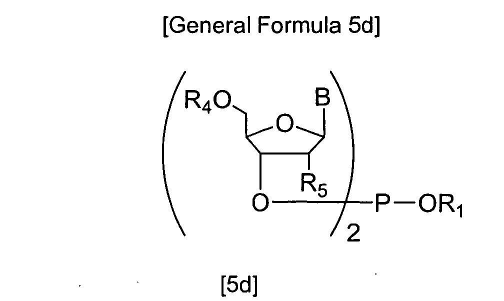 wherein R₁ represents a 2-cyanoethyl group; R₄ represents a 4,4'-dimethoxytrityl group; R₅ represents a hydrogen atom; and B represents N4-benzoyl-1-cytosine.

### Comparative Examples 9 to 10

### Synthesis of N4-benzoyl-5'-O-(4,4'-dimethoxytrityl)-2'-deoxycytidine 3'-O-(2-cyanoethyl N,N-diisopropylphosphoroamidite)

The reaction was conducted in the same manner as in Example 5, except that 0.088 g of tetrazole (0.4 equivalent to the molar number of a raw material: Comparative Example 9) and 0.244 g of pyridinium trifluoroacetate (0.4 equivalent to the molar number of a raw material: Comparative Example 10) were respectively used, instead of 5-phenyl-1 H-tetrazole. The reaction solution after 5 hours was analyzed according to the high performance liquid chromatography (reverse phase column, eluent: water/acetonitrile 4/6 (TEAA 62.5 mM), detection wavelength: 254 nm). The results are shown in Table 4.

**[Table 4]**

| | Example 5 | Comparative Example 9 | Comparative Example 10 |
|---|---|---|---|
| Reaction Selectivity (after 5 hours) | 284 | 177 | 117 |
| Yield (%, after 5 hours) | 99 | 97 | 92 |

The reaction selectivity is the ratio of HPLC area % of the entitled compound to HPLC area % of the by-product represented by the general formula [5d] (HPLC area % of the entitled compound / HPLC area % of the by-product represented by the general formula [5d]). In the formula [5d] of the by-product, R₁ represents a 2-cyanoethyl group, R₄ represents a 4,4'-dimethoxytrityl group, R₅ represents a hydrogen atom and B represents N4-benzoyl-1-cytosine.

## Claims

1. A method for preparing phosphoroamidite with a reagent of a compound represented by the general formula [1], 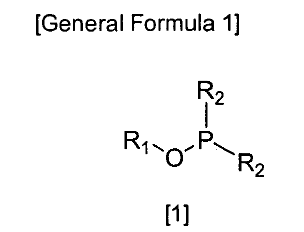 wherein R₁ represents an alkyl group having 1 to 4 carbon atoms, an alkyl group having 1 to 4 carbon atoms substituted by a cyano group or an alkyl group having 1 to 4 carbon atoms substituted by a silyl group; and R₂ represents an amino group substituted by an alkyl group having 2 to 5 carbon atoms or an alicyclic amino group having 4 to 7 carbon atoms,
wherein a substituted tetrazole represented by the general formula [2] is used as a reaction activator, 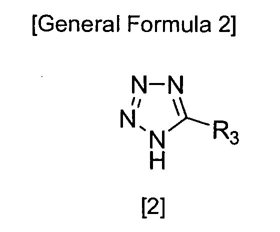 wherein R₃ represents an alicyclic alkyl group having 1 to 6 carbon atoms, an aryl group substituted by an alkyl group having 1 to 4 carbon atoms or an unsubstituted aryl group.

2. The preparation method according to claim 1, wherein phosphoroamidite represented by the general formula [4] is synthesized by using a nucleoside derivative represented by the general formula [3] as a raw material, 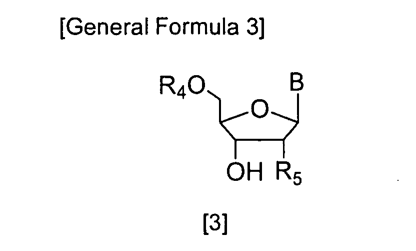 wherein R₄ represents a protecting group of a hydroxyl group; R₅ represents a hydrogen atom, a halogen atom, an alkyl group having 1 to 4 carbon atoms or a substituted hydroxyl group; and B represents a nucleic acid base or a protected nucleic acid base, 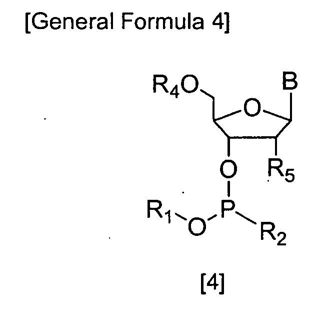 wherein R₁, R₂, R₄, R₅ and B represent the same as those described above.

3. The preparation method according to claim 1 or 2, wherein R₃ in the general formula [2] is a phenyl group.

4. The preparation method according to any one of claims 1 to 3, wherein, in the general formula [1], R₁ is a cyanoethyl group and R₂ is a diisopropylamino group.

5. The preparation method according to any one of claims 2 to 4, wherein, in the general formulae [3] and [4], R₄ is a 4,4'-dimethoxytrityl group, R₅ is a hydrogen atom and B is a 1-thymine group, an N4-benzoyl-1-cytosine group, an N6-benzoyl-9-adenine group or an N2-isobutyryl-9-guanine group.
